(19) Europäisches Patentamt
European Patent Office
Office européen des brevets

(11) Publication number: **0 395 182 A1**

# (12) EUROPEAN PATENT APPLICATION

(21) Application number: **90201067.7**

(51) Int. Cl.[5]: **C07C 229/30, A01N 37/06**

(22) Date of filing: **26.04.90**

(30) Priority: **28.04.89 GB 8909809**

(43) Date of publication of application: **31.10.90 Bulletin 90/44**

(84) Designated Contracting States: **BE CH DE DK ES FR GB IT LI NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Lathbury, David Charles**
**17 Diligent Drive**
**Sittingbourne, Kent ME10 2LQ(GB)**

(74) Representative: **Bennett, David Arthur Horder et al**
**4, York Road**
**London SE1 7NA(GB)**

## (54) Herbicidal compounds.

(57) A compound of the formula

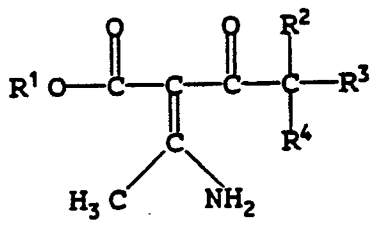

(I)

in which
$R^1$ represents an optionally substituted alkyl, alkenyl, alkynyl, aryl or aralkyl radical;
$R^2$ represents a chlorine atom, a group of the formula
$O-\overset{O}{\overset{\|}{C}}-R^5$, wherein $R^5$ represents a hydrogen atom or an optionally substituted alkyl, alkoxy, alkoxycarbonyl, aryl, aryloxy, aralkoxy, aryloxyalkyl or amino radical,
or
a group of the formula $O-R^6$ wherein $R^6$ represents an optionally substituted heterocyclic ring of which at least the ring member bonded to the oxygen atom or one of the adjacent ring members is a hetero atom;
and
one of $R^3$ and $R^4$ represents a hydrogen atom and the other represents an alkyl radical;
The invention also provides processes for the preparation of compounds of formula I, herbicidal compositions containing them and their use as herbicides.

EP 0 395 182 A1

# HERBICIDAL COMPOUNDS

The present invention relates to derivatives of 3-aminobutenoic acid, also known as 3-aminocrotonic acid.

Many aminobutenoic acid derivatives are known and most often find use in the preparation of a wide range of chemical compounds, often heterocyclic compounds which are themselves intermediates for or compounds having biological activity.

Berner et al in Monatshefte für Chemie (1977) 108, 915-927, concerned with the synthesis of 3-alkoxypyrromethenes describes, inter alia, the preparation of intermediate pyrrolinone derivatives from ethyl 2-(2-chloracetyl)-3-aminocrotonate. The preparation of the same butenoate compound has been reviewed in Bull Soc. Chim. Belg. 90(1), 75-82 by Shabana et al. In neither document is any biological use suggested for the 3-aminobutenoate.

Also, European Patent Specification No. 245130A discloses herbicidally active pyridinecarboxylic acid derivatives and, as intermediates, various 3-amino-4,4,4-trifluorobutenoates. No biological activity of any kind is suggested for those intermediates.

German Offenlegungsschrift No. 23 36 723 discloses a large group of compounds as having herbicidal activity, which group includes alkylated-amino-propenoate and -butenoate derivatives with mono- or di-substitution of the amino group, though specific exemplification of herbicidal properties is only provided for the propenoate derivatives.

A class of 3- (unsubstituted amino)butenoates has now been found which possess valuable herbicidal activity.

Accordingly, the present invention provides a compound of the general formula

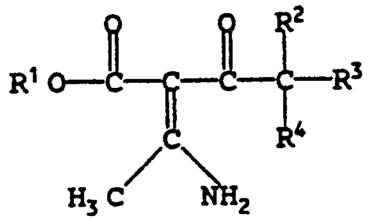

(I)

in which

$R^1$ represents an optionally substituted alkyl, alkenyl, alkynyl, aryl or aralkyl radical;

$R^2$ represents a chlorine atom,

a group of the general formula

$O - \overset{O}{\overset{\|}{C}} - R^5$, wherein $R^5$ represents a hydrogen atom or an optionally substituted alkyl, alkoxy, alkoxycarbonyl, aryl, aralkyl, aryloxy, aralkoxy, aryloxyalkyl or amino radical,

or

a group of the general formula $O-R^6$

wherein $R^6$ represents an optionally substituted heterocyclic ring of which at least the ring member bonded to the oxygen atom or one of the two adjacent ring members is a hetero atom; and

one of $R^3$ and $R^4$, represents a hydrogen atom and the other represents an alkyl radical.

An alkyl, alkenyl or alkynyl radical, or an alkyl moiety in an alkoxy or aralkyl radical, may be a straight or branched chain radical. Suitably an alkyl radical or moiety has from 1 to 12 carbon atoms, preferably from 1 to 6, especially from 1 to 4, carbon atoms. Alkenyl and alkynyl radicals suitably have from 2 to 12 carbon atoms, preferably from 2 to 6, especially from 2 to 4, carbon atoms.

An aryl radical, or an aryl moiety in an aralkyl or aryloxy radical, may be a single or fused carbocyclic ring system having from 6 to 10 ring members. Suitably an aryl radical or moiety comprises a single ring system and preferably is a phenyl ring.

A heterocyclic radical is suitably a single or fused, saturated or unsaturated ring system having from 5 to 10 ring members of which from 1 to 3 ring members may be hetero atoms selected from oxygen, nitrogen and sulphur atoms, with either at least one hetero atom being adjacent, or alpha to, the ether-carbon ring member or the ring member bonded to the ether-oxygen atom being a suitable hetero atom, for example a nitrogen atom.

Radicals represented by the symbols $R^1$, $R^5$ and $R^6$ may be unsubstituted or substituted. Where substituents are present, the substituent groups may be any of those customarily employed in the modification and/or development of pesticidal compounds and are especially substituents that maintain or enhance the herbicidal activity associated with the butenoate compounds of the present invention, or influence persistence of action, soil or plant penetration, or any other desirable property of such herbicidal compounds. There may be one or more of the same or different substituents present in each radical. Suitable examples of substituent groups include halogen atoms, especially fluorine, chlorine or bromine atoms, nitro groups, cyano groups, free or modified hydroxy groups, free or modified carboxy groups, alkyl groups unsubstituted or substituted by one or more halogen atoms, especially fluorine atoms, and aryl groups unsubstituted or substituted by one or more halogen atoms, especially chlorine atoms, or nitro, cyano or haloalkyl groups. Modified hydroxy and carboxy groups are to be understood to be groups derived from hydroxy or carboxy groups by, for example, etherification, esterification, acylation or salt formation, as appropriate for the group in question.

. An alkyl radical, when present as a substituent, preferably has from 1 to 4 carbon atoms, especially 1 or 2 carbon atoms, and as a halogen-substituted alkyl group, a trifluoromethyl group may especially be mentioned. An aryl radical, when present as a substituent, is preferably a phenyl group, and as a halogen-substituted aryl group, a 4-chlorophenyl group may especially be mentioned.

Suitable examples of radical $R^1$ include optionally substituted $C_{1-6}$ alkyl, $C_{2-6}$ alkenyl, $C_{2-6}$ alkynyl, phenyl and aryl($C_{1-6}$)alkyl groups. Preferably, the group $R^1$ is selected from $C_{1-4}$ alkyl, $C_{2-4}$ alkenyl, phenyl and phen($C_{1-4}$)alkyl groups. Preferably the group $R^1$ is a methyl, ethyl, propyl, butyl, allyl or benzyl group, and $R^1$ is especially an unsubstituted methyl, ethyl, <u>tert</u> butyl, allyl or benzyl group.

Suitably one of the radicals $R^3$ and $R^4$ represents a hydrogen atom and the other represents a $C_{1-6}$ alkyl radical. Preferably one of the radicals $R^3$ and $R^4$ represents a hydrogen atom and the other represents a $C_{1-4}$ alkyl, especially a methyl group.

The radical $R^2$ may represent a chlorine atom, a group

$O-\overset{\overset{O}{\|}}{C}-R^5$ or a group O-$R^6$. Suitable examples of the group

$O-\overset{\overset{O}{\|}}{C}-R^5$

include optionally substituted formyl, ($C_{1-6}$)alkyl carbonyl, ($C_{1-6}$)alkoxycarbonyl, ($C_{1-6}$)-alkoxycarbonylcarbonyl, arylcarbonyl, or aryl ($C_{1-6}$)alkylcarbonyl, aryloxycarbonyl, aryloxy($C_{1-6}$)-alkylcarbonyl, carbamoyl and arylcarbamoyl groups. Suitable examples of the radical $R^6$ include optionally substituted tetrahydrofuran-2-yl, tetrahydropyran-2-yl, pyrid-1 or 2-yl, isobenzylfuran- 1-yl, chroman-2-yl, chromen-2-yl, isoxazol-2 or 3-yl, furazan-2 or 3-yl and morpholin-2, 3- or 4-yl.

Preferably the radical $R^2$ represents a chlorine atom; a group

$O-\overset{\overset{O}{\|}}{C}-R^5$

in which $R^5$ represents a hydrogen atom, a $C_{1-4}$ alkyl, for example methyl, ethyl or propyl, group, a $C_{1-4}$ alkoxy group, for example a methoxy group, a $C_{1-4}$ alkoxycarbonyl group, for example a methoxycarbonyl group, an aryl group, for example a phenyl group, an aryl ($C_{1-4}$)alkyl group, for example a benzyl group, an aryloxy group, for example a phenoxy group, an aryl ($C_{1-4}$)alkoxy group, for example a benzyloxy group, or an aryloxy($C_{1-4}$)alkyl group, for example a phenoxymethyl group, each of which being optionally substituted, or an amino group, preferably mono- or di-substituted, for example a phenylamino group; or a group O-$R^6$ in which $R^6$ represents an optionally substituted tetrahydropyran-2-yl group.

It will be appreciated that the compounds of the present invention have an assymetric carbon atom and therefore that compounds of the general formula I may exist in different stereoisomeric forms. Furthermore, the compounds of the general formula I may exist in both <u>E</u> and <u>Z</u> stereoisomeric form. Therefore the compounds of the recent invention may exist in four stereoisomeric forms. The present invention encompasses all stereoisomers of the compounds of general formula I whether free from other isomers or admixed with other isomers in any proportion, and thus includes, for example, racemic mixtures of enantiomers.

The present invention further provides a process for the preparation of a compound of the general formula I which comprises either a) reacting at reduced temperature, a compound of the general formula

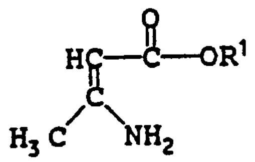

(II)

in which $R^1$ is as hereinbefore defined, with a compound of the general formula

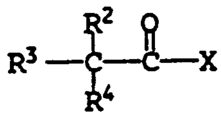

(III)

in which $R^2$, $R^3$ and $R^4$ are as hereinbefore defined, and X represents a leaving group,
or
b) reducing a compound of the general formula

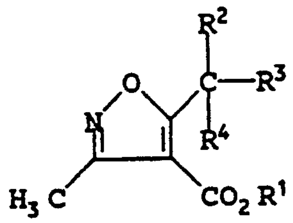

(IV)

in which $R^1$, $R^2$, $R^3$ and $R^4$ are as hereinbefore defined.

A leaving group represented by X is any group that will, under the reaction conditions, cleave from the starting material thus promoting reaction at a specified site. The group X in a compound of general formula III may be, for example, a halogen atom, preferably a chlorine or bromine atom, but is especially a chlorine atom.

The reaction a) is suitably carried out under basic conditions, for example in the presence of an alkali metal or alkaline earth metal hydroxide or bicarbonate, or a tertiary amine, suitably triethylamine or pyridine. Also the reaction a) is suitably carried out in a solvent inert to the reaction media, which solvent is conveniently an organic solvent, for example diethyl ether or a chlorinated hydrocarbon, e.g. dichloromethane.

Suitably the reaction of compounds II and III is carried out at a temperature below 0° C, preferably at a temperature in the range of from -50 to -80° C, especially between -70 and -80° C, and allowed to warm, while the reaction is occurring, to ambient temperature. Conveniently substantially equimolar amounts of reactants II and III may be utilised.

The reaction b) is suitably carried out under reducing conditions usual in the art for such reactions. Preferably the reduction is effected by conventional hydrogenation techniques, especially by catalytic hydrogenation using hydrogen gas, or a compound or mixture of compounds that generate hydrogen in situ, in conjunction with a platinum or palladium on carbon catalyst. For the preparation of compounds of general formula I from a halogen-containing compound, for example a compound IV in which $R^2$ represents a chlorine atom, it is necessary to ensure that the catalyst utilised does not react with the starting material and therefore, for example, a platinum oxide catalyst should be employed in preference to a palladium/carbon catalyst.

It is preferred that for the preparation of compounds of general formula I in which $R^2$ represents a chlorine atom, the process a) is employed and that process b) is used for the preparation of compounds of the present invention in which $R^2$ is other than a chlorine atom.

The resulting compound of the present invention may, if desired, be isolated and purified using conventional techniques, for example solvent extraction, evaporation and then recrystallisation or chromatography on silica.

Compounds of general formula II may be prepared using methods known to those skilled in the art.

Compounds of general formula III are either known compounds or may prepared using techniques

described in the literature.

Compounds of general formula IV in which $R^2$ is other than a chlorine atom may be prepared from the corresponding isoxazole

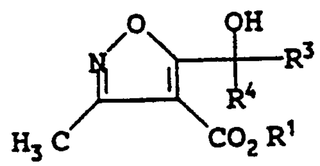

(V)

in which $R^1$, $R^3$ and $R^4$ are as hereinbefore defined, by conventional esterification or acylation procedures, for example by reaction with a compound of the general formula $R^5COHal$, in which $R^5$ is as hereinbefore defined, but preferably is other than an amino group, and Hal represents a halogen atom, in a suitable base, for example triethylamine, or by reaction with an isocyanate of the general formula $R^7CO$, in which $R^7$ represents an optionally substituted imino group, suitably at reduced temperature.

Compounds of general formula V are either known or may be prepared using techniques described in the literature, for example from the reduction of a corresponding isoxazole which, in place of the OH group, has a tetrahydropyran-2-yloxy group. Such isoxazoles are suitable for preparing directly, by reduction, compounds of the general formula I in which $R^2$ represents a tetrahydropyran-2-yloxy group, and are also either known or preparable by appropriate conventional techniques.

The present invention further provides the use of a compound of the present invention as a herbicide.

The present invention additionally provides a herbicidal composition which comprises a compound of the invention in association with a carrier. More than one carrier may be present in a composition of the invention and suitably at least one carrier is a surface-active agent.

Additionally, the present invention provides a method of combating undesired plant growth at a locus by treating the locus with a compound or composition of the invention. Application to the locus may be pre-emergence or post-emergence. The dosage of active ingredient applied is suitably in the range of from 0.01 to 10 kg/ha, preferably from 0.05 to 5 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol

or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$ -10% w of active ingredient. Granules are usually prepared to have a size between 10 and 100 BS mesh (1.676 - 0.152 mm), and may be manufactured by agglomeration or impregnation techniques. Generally, granules will contain $\frac{1}{2}$ -75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The following Examples illustrate the present invention.

Example 1

3-Amino-2-(2-chloropropanoyl)-2-butenoic acid methyl ester

Over the period of 1 hour 1.2g (0.0094moles) of 2-chloropropionylchloride in 20ml dry diethyl ether was added to a solution of 1g (8.6mmoles) of methylaminocrotonate in 50ml and 0.74ml pyridine at -78° C. The reaction mixture was allowed to warm to room temperature over the course of 1 hour, diluted with a 2:1, by volume, mixture of diethyl ether and dichloromethane, washed with 20ml of water, dried over magnesium sulphate and the solvent removed. Following purification by silica gel chromatography using 1:1, by volume, diethyl ether/hexane as eluant, 0.48g of the title compound was obtained as a white solid.

Analysis (%):

Calc. C 46.8 H 5.8 N 6.8

Found C 46.0 H 5.9 N 7.2

H'NMR $CDCl_3$ (δ): 11.1brs 1H, 5.92brs 1H, 5.2q 1H, 3.75s 3H, 2.25brs 3H, 1.6d 3H.

Example 2

3-Amino-2-(2-acetoxypropanoyl)-2 butenoic acid methyl ester

a) <u>2-Butynol tetrahydropyran ether</u>

10g of Butyn-2-ol and 15.6g of dihydropyran were mixed and then cooled to 0°C. 1 drop of concentrated sulphuric acid was added to the mixture which was then stirred for 20 minutes while being maintained at 0°C. Subsequently the mixture was warmed to room temperature and stirred for 2 hours, then diluted with 80ml of diethyl ether, washed with 15ml of sodium bicarbonate solution and dried over sodium sulphate. The solvent was removed in vacuo and the product purified by distillation to yield 18g of 2-butynol tetrahydropyran ether as a clear oil.
Analysis (%):
Calc. C 70.1 H 9.1
Found C 67.2 H 8.7

b) <u>4-(Tetrahydropyran-2-yl)-oxy-2-butynoic acid methyl ester</u>

A solution of 4.7ml of n-butyl lithium in hexane was added dropwise and with rapid stirring to a solution of 1g of the acetylene of a) above in 50ml of dry tetrahydrofuran at -78°C under nitrogen. The reaction mixture was stirred for 30 minutes at -70°C and then added to a solution of 0.85g of methyl chloroformate in 25ml of dry tetrahydrofuran over a period of 5 minutes. The mixture was then warmed to room temperature, diluted with 80ml of diethyl ether, washed with 25ml of water, dried over magnesium sulphate and the solvent removed. On purification by distillation, 1g of the title compound was obtained.
Analysis (%):
Calc. C 62.2 H 7.5
Found C 61.2 H 7.8

c) <u>3-Methyl-5-[1-(tetrahydropyran-2-yl)-oxy]-ethyl isoxazole-4-carboxylic acid methyl ester</u>

A solution of 0.68g of nitroethane and 0.1g of triethylamine in 20ml of benzene was added dropwise over a period of 2 hours to a solution of 0.83g of the compound prepared in b) above and 1.96g of phenyl isocyanate in 25ml of dry benzene. The reaction mixture was stirred at room temperature for 20 hours, then diluted with 50ml of a 1:1 (by volume) mixture of diethyl ether and petroleum ether, filtered and the solvent removed. Following silica gel chromatography using diethyl ether/hexane (1:4 by volume) as eluant, 0.81g of the isoxazole was obtained as a colourless oil.
Analysis (%):
Calc. C 57.9 H 7.1 N 5.2
Found C 58.2 H 7.2 N 5.6

d) <u>3-Methyl-5-(1-hydroxyethyl)-isoxazole-4-carboxylic acid methyl ester</u>

0.1g of p-toluene sulphonic acid was added to a solution of 2g of the compound prepared as in c) above in 25ml of methanol. Following stirring at room temperature for 3 hours, the reaction mixture was diluted with 25ml of diethyl ether, washed twice with 5ml aliquots of sodium carbonate solution, dried over magnesium sulphate and the solvent removed. After purification by silica gel chromatography using diethyl ether/hexane (1:4 by volume) as eluant, 1.3g of the title compound was obtained as a clear oil.
Analysis (%):
Calc. C 51.9 H 5.9 N 7.5
Found C 51.6 H 6.1 N 7.4

e) <u>3-Methyl-5-(1-acetoxyethyl)-isoxazole-4-carboxylic acid methyl ester</u>

1g of triethylamine was added to a solution of 1g of the product of d) above in 25ml of dry diethyl ether and the mixture cooled to 0°C. 0.5g of acetyl chloride in 1ml diethyl ether was added dropwise, with rapid stirring, to the solution over a period of 5 minutes. The reaction mixture was warmed to room temperature and stirred for 1 hour. The mixture was then washed twice using two 10ml aliquots of 2N hydrochloric acid, dried over magnesium sulphate and the solvent removed. Following purification by silica gel chromatography using diethyl ether/hexane (1:4 by volume), 1.1g of the title compound was obtained as a colourless oil.
Analysis (%):
Calc. C 52.8 H 5.7 N 6.1
Found C 53.3 H 5.9 N 6.3

f) 3-Amino-2-(2-acetoxypropanoyl)-2-butenoic acid methyl ester

200mg of 10% by weight palladium on carbon catalyst was added to a solution of 0.85g of the product of e) above in 10ml of methanol and the mixture stirred under hydrogen overnight. Following dilution with 50ml of diethyl ether, filtration through Florisil ("Florisil" is a trade mark) and removal of the solvent, the product was purified by silica gel chromatography using diethyl ether as eluant to yield 0.78g of the title compound.
Melting point: 102-104° C
Analysis (%):
Calc. C 52.4 H 6.5 N 6.1
Found C 52.1 H 6.5 N 6.4
H′nmr $CDCl_3$ (δ): 11.2brs 1H, 5.9brs 1H, 5.66q 1H, 3.75s 3H, 2.25s 3H, 2.1s 3H, 1.4d 3H.

Further compounds of general formula I were prepared in analogous manner to the procedures described in Example 1 and Example 2. Details are given in Table 1 below.

Table 1

| Ex. No. | $R^1$ | $R^2$ | $R^3$ | $R^4$ | Identification data: Analysis (%) (Calc.; Found) C | H | N | $H^1$nmr $CDCl_3$ (δ) |
|---|---|---|---|---|---|---|---|---|
| 3 | $C_2H_5$ | Cl | $CH_3$ | H | 49.4<br>50.6 | 6.4<br>6.5 | 6.4<br>6.3 | 11.1brs 1H, 5.85brs 1H, 5.23q 1H, 4.25q 2H, 2.27s 3H, 1.6d 3H, 1.42+3H |
| 4 | $CH_2=CHCH_2$ | Cl | $CH_3$ | H | 51.9<br>51.6 | 6.1<br>6.0 | 6.1<br>6.1 | 11.13brs 1H, 6.1-5.7m 2H, 5.4-5.15m 3H, 4.65brd 2H, 2.25brs 3H, 1.6d 3H |
| 5 | $CH_2C_6H_5$ | Cl | $CH_3$ | H | 59.2<br>58.6 | 5.7<br>5.5 | 5.0<br>4.9 | 11.1brs 1H, 7.45-7.3m 5H, 5.75brs 1H, 5.24-5.15m 3H, 2.22s 3H, 1.55d 3H |
| 6 | <u>t</u>-$C_4H_9$ | Cl | $CH_3$ | H | 53.4<br>51.7 | 7.2<br>7.1 | 5.6<br>5.3 | 11.0brs 1H, 5.65brs 1H, 5.2q 1H, 2.23s 3H, 1.62d 3H, 1.5s 9H |

| Ex. No. | $R^1$ | $R^2$ | $R^3$ | $R^4$ | mp (°C) | Identification data: Analysis (%) (Calc. Found) C | H | N | $H^1$nmr $CDCl_3$ (δ) |
|---|---|---|---|---|---|---|---|---|---|
| 7 | $CH_3$ | OCHO | $CH_3$ | H | 105-108 | 50.2<br>49.4 | 6.05<br>5.9 | 6.51<br>6.7 | 11.3 brs 1H, 8.05s 1H, 5.85m 2H, 3.75s 3H, 2,3s 3H, 1.45d 3H |
| 8 | $CH_3$ | $OC(O)C_6H_5$ | $CH_3$ | H | 94-96 | 61.8<br>61.3 | 5.8<br>5.8 | 4.8<br>5.0 | 11.2brs 1H, 8.05-7.4m 5H, 5.9q 1H, 5.8brs 1H, 3.7s 3H, 2.28s 3H, 1.55d 3H |
| 9 | $CH_3$ | $OC(O)OCH_3$ | $CH_3$ | H | 107-110 | 48.9<br>49.1 | 6.1<br>6.7 | 5.7<br>5.7 | 11.25brs 1H, 5.92brs 1H, 5.65q 1H, 3.78s 3H, 3.75s 3H, 2.27s 3H, 1.45d 3H |
| 10 | $CH_3$ | $OC(O)CH_2O$ 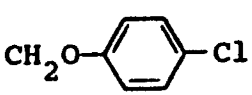 | $CH_3$ | H | oil | 54.0<br>54.0 | 5.9<br>6.0 | 3.9<br>3.8 | 11.25br s 1H, 7.25-6.8m 4H, 5.9-5.75m 2H, 4.7s 2H, 3.75s 3H, 1.45d 3H |

| Ex. No. | $R^1$ | $R^2$ | $R^3$ | $R^4$ | mp (°C) | Identification data: Analysis (%) (Calc. Found) C | H | N | $H^1$nmr $CDCl_3$ (δ) |
|---|---|---|---|---|---|---|---|---|---|
| 11 | $CH_3$ | $OC(O)C_3H_7$ | $CH_3$ | H | 107-110 | 56.1<br>56.0 | 7.3<br>7.2 | 5.4<br>5.7 | 11.2brs 1H, 5.85brs 1H, 5.65q 1H, 3.7s 3H, 2.35+2H, 2.25s 3H, 1.65m 2H, 1.4d 3H, 0.95+3H |
| 12 | $CH_3$ | $OC(O)NHC_6H_5$ | $CH_3$ | H | 117-120 | 58.8<br>58.4 | 5.8<br>6.0 | 9.1<br>9.3 | 10.5brs 1H, 8.7brs 1H, 7.8brs 1H, 7.1-6.5m 5H, 5.3q 1H, 3.3s 3H, 1.8s 3H, 1.0d 3H [in $D^6$DMSO not $CDCl_3$] |
| 13 | $CH_3$ | $OC(O)C(O)OCH_3$ | $CH_3$ | H | 103-107 | 48.3<br>48.0 | 4.8<br>5.2 | 5.1<br>4.7 | 11.25brs 1H, 6.1brs 1H, 5.9q 1H, 3.9s 3H, 3.75s 3H, 2.3s 3H, 1.5d 3H. |

Example 14

3-Amino-2-(2-(tetrahydropyran-2-yloxy)propanoyl)-2-butenoic acid methyl ester

0.2g of 3-methyl-5-[1-(tetrahydropyran-2-yl)oxy]-ethyl isoxazole-4-carboxylic acid methyl ester prepared as in Example 2c, was subjected to hydrogenation using a palladium on carbon catalyst as in Example 2f) to give 0.18g of the title compound after chromatography on silica using diethyl ether/hexane (1:1 by volume) as eluant.
Analysis (%):
Calc. C 57.5 H 8.1 N 5.1
Found C 57.4 H 7.8 N 5.2
H'NMR $CDCl_3$ (δ): 11.15brs 1H, 5.55brs 1H, 4.8-4.5m 2H, 3.93-3.35m 5H, 2.2brs 3H, 1.85-1.3m 9H

Example 15

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in the following Table in which the compounds are identified by reference to the preceding Examples. Absence of a numeral in the Table indicates a zero rating, an asterisk indicates that no result was obtained.

Table 2

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 6 | 6 | 6 | 6 | 7 | 4 | 6 | 5 | 5 | 5 | 5 | 6 | 4 | 6 | 5 | 5 | 5 | 6 | 6 | 9 | 6 | 8 | 7 | 8 | 6 |
| | | | | | | | | | 1 | 3 | 3 | 4 | 3 | 6 | 3 | 3 | 4 | 3 | | 5 | 4 | 6 | 3 | 2 | |
| 2 | 6 | 6 | 6 | 6 | 7 | 6 | 6 | 7 | 5 | 6 | 6 | 7 | 6 | 7 | 6 | 6 | 7 | 9 | 8 | 9 | 6 | 8 | 9 | 9 | 8 |
| | | | | | | | | | 1 | 3 | 3 | 4 | 4 | 6 | 4 | 5 | 6 | 5 | 1 | 4 | 4 | 7 | 4 | 7 | 4 |
| 3 | 7 | 6 | 7 | 6 | 7 | 6 | 6 | 7 | 5 | | | 4 | 4 | 6 | 6 | 4 | 7 | 5 | 6 | 6 | 6 | 6 | 5 | 6 | 5 |
| | | | | | | | | | 1 | | | 1 | 2 | 5 | 2 | 1 | 4 | | | 1 | 4 | 4 | 1 | 1 | |
| 4 | 7 | 7 | 7 | 6 | 7 | 6 | 6 | 8 | 5 | 3 | 4 | 7 | 5 | 6 | 5 | 5 | 7 | 6 | 6 | 7 | 6 | 7 | 6 | 6 | 7 |
| | | | | | | | | | 1 | 1 | | 2 | 2 | 5 | 2 | 2 | 4 | 1 | 1 | 1 | 5 | 5 | 2 | 3 | |
| 5 | 7 | 7 | 7 | 6 | 7 | 6 | 6 | 7 | 5 | 4 | 5 | 6 | 5 | 6 | 6 | 4 | 6 | 7 | 6 | 7 | 6 | 8 | 5 | 7 | 6 |
| | | | | | | | | | 1 | 1 | | 1 | 2 | 5 | 4 | 1 | 4 | 2 | | 2 | 5 | 6 | 2 | 3 | |

Table 2 (continued)

| Compound of Ex. No. | Soil drench 10/kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 6 | 7 | 7 | 7 | 6 | 7 | 6 | 6 | 8 | 5 | 5 | 6 | 7 | 5 | 7 | 6 | 6 | 7 | 7 | 7 | 8 | 7 | 8 | 7 | 8 | 8 |
| | | | | | | | | | 1 | 1 | 1 | 4 | 4 | 5 | 4 | 3 | 6 | 3 | 4 | 4 | 5 | 6 | 3 | 4 | 4 |
| 7 | 7 | 7 | 7 | 7 | 8 | 7 | 6 | 8 | 5 | 4 | 5 | 6 | 6 | 8 | 7 | 6 | 7 | 6 | 7 | 8 | 6 | 8 | 8 | 9 | 7 |
| | | | | | | | | | 1 | | | 1 | 3 | 7 | 6 | 5 | 5 | | 2 | | 4 | 6 | 3 | 4 | |
| 8 | 7 | 7 | 7 | 7 | 7 | 7 | 7 | 7 | 5 | 2 | 6 | 5 | 6 | 7 | 5 | 7 | 7 | 6 | 6 | 7 | 6 | 7 | 7 | 8 | 8 |
| | | | | | | | | | 1 | 1 | 1 | 1 | 5 | 6 | 3 | 6 | 5 | 2 | 5 | 3 | 4 | 4 | 3 | 4 | |
| 9 | 6 | 6 | 6 | 7 | 8 | 7 | 6 | 6 | 5 | 4 | 5 | 4 | 6 | 7 | 5 | 5 | 7 | 6 | 7 | 8 | 5 | * | * | 8 | 8 |
| | | | | | | | | | 1 | | | | | 7 | 3 | 4 | 5 | | 1 | 2 | 2 | * | * | 7 | 0 |

Table 2 (continued)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 10 | 6 | 5 | 7 | 6 | 8 | 9 | 9 | 7 | 5 | 5 | 2 | 7 | 6 | 8 | 7 | 8 | 8 | 7 | 7 | 8 | 7 | 8 | 9 | 9 | 8 |
| | | | | | | | | | 1 | 2 | | 1 | 4 | 7 | 6 | 5 | 6 | 2 | 6 | 7 | 3 | 7 | 8 | 8 | 7 |
| 11 | 7 | 7 | 7 | 7 | 8 | 7 | 6 | 7 | 5 | 2 | 4 | 4 | 5 | 7 | 6 | 6 | 6 | 7 | 8 | 9 | 6 | 8 | 8 | 8 | 7 |
| | | | | | | | | | 1 | | | | 2 | 6 | 4 | 4 | 4 | 1 | 5 | 7 | 4 | 6 | 4 | 4 | 1 |
| 12 | | | | | 3 | | | | 5 | | | | 2 | 4 | 3 | 0 | 2 | 2 | | | | | | | |
| | | | | | | | | | 1 | | | | | 3 | | | | | | | | | | | |
| 13 | 6 | 5 | 7 | 6 | 7 | 6 | 6 | 7 | 5 | 5 | 5 | 5 | 5 | 7 | 6 | 6 | 6 | 5 | 6 | 8 | 5 | 7 | 8 | 9 | 6 |
| | | | | | | | | | 1 | 3 | 2 | 2 | 4 | 7 | 4 | 5 | 5 | 1 | 2 | 3 | 3 | 5 | 3 | 4 | 0 |
| 14 | 5 | 5 | 4 | 5 | 6 | 4 | 5 | 5 | 5 | 2 | | 2 | 1 | 6 | 4 | 3 | 3 | | 2 | 1 | 3 | 5 | 2 | 4 | 1 |
| | | | | | | | | | 1 | | | 1 | | 4 | 2 | 1 | | | | | | 2 | | | |

## Claims

1. A compound of the general formula

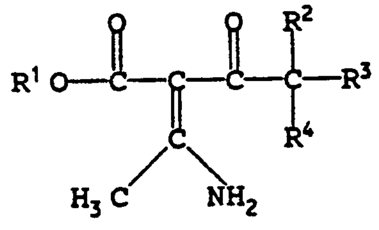

(I)

in which
$R^1$ represents an optionally substituted alkyl, alkenyl, alkynyl, aryl or aralkyl radical;
$R^2$ represents a chlorine atom, a group of the general formula
$O-\overset{\overset{O}{\|}}{C}-R^5$, wherein $R^5$ represents a hydrogen atom or an optionally substituted alkyl, alkoxy, alkoxycarbonyl, aryl, aralkyl, aryloxy, aralkoxy, aryloxyalkyl or amino radical,
or
a group of the general formula $O-R^6$
wherein $R^6$ represents an optionally substituted heterocyclic ring of which at least the ring member bonded to the oxygen atom or one of the two adjacent ring members is a hetero atom; and
one of $R^3$ and $R^4$, represents a hydrogen atom and the other represents an alkyl radical.

2. A compound as claimed in claim 1, wherein $R^1$ represents an optionally substituted $C_{1-4}$ alkyl, $C_{2-4}$ alkenyl, $C_{2-4}$ alkynyl, phenyl or phen($C_{1-4}$)alkyl group.

3. A compound as claimed in claim 1 or claim 2, wherein one of the radicals $R^3$ and $R^4$ represents a hydrogen atom and the other represents a $C_{1-4}$ alkyl group.

4. A compound as claimed in any one of claims 1 to 3, wherein $R^2$ represents a chlorine atom; a group $O-\overset{\overset{O}{\|}}{C}-R^5$ in which $R^5$ represents a hydrogen atom, a $C_{1-4}$alkyl group, a $C_{1-4}$ alkoxy group, a $C_{1-4}$alkoxycarbonyl group, an aryl group, an aryl($C_{1-4}$)alkyl group, an aryloxy group, an aryl ($C_{1-4}$)alkoxy group an aryloxy($C_{1-4}$)alkyl group each of which being optionally substituted, or an unsubstituted or mono- or di-substituted amino group; or a group $O-R^6$ in which $R^6$ represents an optionally substituted tetrahydropyran-2-yl group.

5. A process for the preparation of a compound of general formula I claimed in claim 1, which comprises either

a) reacting, at reduced temperature, a compound of the general formula

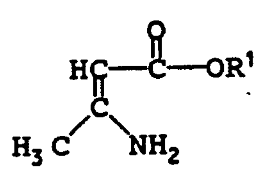

(II)

in which $R^1$ is as defined in claim 1, with a compound of the general formula

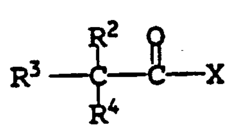

(III)

in which $R^2$, $R^3$ and $R^4$ are as defined in claim 1, and X represents a leaving group, or

b) reducing a compound of the general formula

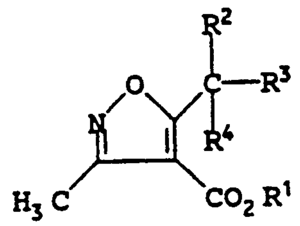

(IV)

in which $R^1$, $R^2$, $R^3$ and $R^4$ are as defined in claim 1.

6. A process as claimed in claim 5, wherein, in process variant a), the reaction is carried out at a temperature in the range of from -50 to -80 °C.

7. A herbicidal composition which comprises a compound as claimed in any one of claims 1 to 4, in association with a carrier.

8. A method of combating undesired plant growth at a locus, which comprises treating the locus with a compound as claimed in any one of claims 1 to 4, or with a composition as claimed in claim 7.

9. The use of a compound as claimed in any one of claims 1 to 4, as a herbicide.

European Patent Office

# EUROPEAN SEARCH REPORT

Application Number

EP 90 20 1067
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | BULLETIN DES SOCIETES CHIMIQUES BELGES. vol. 90, no. 1, 1981, OXFORD GB pages 75 - 82; SHABANA R. et al.: "ENAMINE CHEMISTRY,PART XXI." * pages 77 - 78 * | 1 | C07C229/30<br>A01N37/06 |
| A,D | MONATSHEFTE FUR CHEMIE. vol. 108, no. 4, 1977, WIEN AT pages 915 - 927; BERNER, HEINZ et al.: "CHEMIE DER METACYCLOPRODIGIOSINE." * page 917 * | 1 | |
| A | JOURNAL OF ORGANIC CHEMISTRY. vol. 45, no. 23, 07 November 1980, EASTON US pages 4608 - 4611; FREDERICK E. WARD et al.: "REACTION OF 3-AMINO-2-BENZOYLCROTONATE ESTERS WITH PHOSGENE." * page 4608, right-hand column * | 5 | |
| A | TETRAHEDRON, (INCL. TETRAHEDRON REPORTS) vol. 25, no. 2, 1969, OXFORD GB pages 389 - 395; D.N.McGREGOR et al.: "SYNTHESIS OF ISOTHIAZOLES." * the whole document * | 5 | TECHNICAL FIELDS SEARCHED (Int. Cl.5 )<br>C07C |
| A | CHEMICAL ABSTRACTS, vol. 90, no. 19, 07 May 1979 Columbus, Ohio, USA YAKIMOVICH,S.I.et al.: "REACTION OF IMINES OF BETA-KETO ESTERS WITH TRIFLUOROACETIC ANHYDRIDE." page 554; right-hand column; ref. no. 151536P * abstract * | 1, 5 | |

-/--

The present search report has been drawn up for all claims

| Place of search | Date of completion of the search | Examiner |
|---|---|---|
| BERLIN | 18 JUNE 1990 | RUFET, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 90 20 1067
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 72, no. 11, 16 March 1970<br>Columbus, Ohio, USA<br>KATO TETSUZO et al.: "KETENE AND ITS DERIVATIVES.XXXIII.ACETYLATION OF ETHYL 3-AMINOCROTONATE."<br>page 346; right-hand column; ref. no. 54676E<br>* abstract * | 1, 5 | |
| A,D | DE-A-2336723 (BAYER AG.)<br>* the whole document * | 1, 7 | |
| A | EP-A-135838 (BASF)<br>* claims 1, 7 * | 1, 7 | |

TECHNICAL FIELDS SEARCHED (Int. Cl.5 )

The present search report has been drawn up for all claims

| Place of search | Date of completion of the search | Examiner |
|---|---|---|
| BERLIN | 18 JUNE 1990 | RUFET, J |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)